# EUROPEAN PATENT SPECIFICATION

(11) **EP 4 278 994 B1**
(45) Date of publication and mention of the grant of the patent: **30.07.2025**
(21) Application number: 23172900.5
(22) Date of filing: 11.05.2023
(51) Int. Cl.: A61B 17/32, A61B 17/00

(54) **SURGICAL TISSUE SHAVER WITH ANGLED TEETH**
CHIRURGISCHER GEWEBERASIERER MIT ABGEWINKELTEN ZÄHNEN
RASOIR CHIRURGICAL POUR TISSUS AVEC DENTS INCLINÉES

(30) Priority: 18.05.2022 US 202217747695
(43) Date of publication of application: 22.11.2023
(73) Proprietor: Lenkbar, LLC, Naples, FL 34104 (US)
(72) Inventor: PAPENFUSS, Erik H., Naples, FL, 34104 (US)
(74) Representative: Kransell & Wennborg KB

(56) References cited:
- US-A1- 2005 065 538
- US-A1- 2006 196 038
- US-A1- 2019 038 305

## Description

### Background

Surgical tissue shavers are devices that remove tissue from a patient's body. A typical tissue shaver has a cutting head formed by concentric inner and outer tubes or shafts. The concentric shafts are positioned next to tissue to be removed, and moved relative to each other to generate a cutting action to remove the tissue.

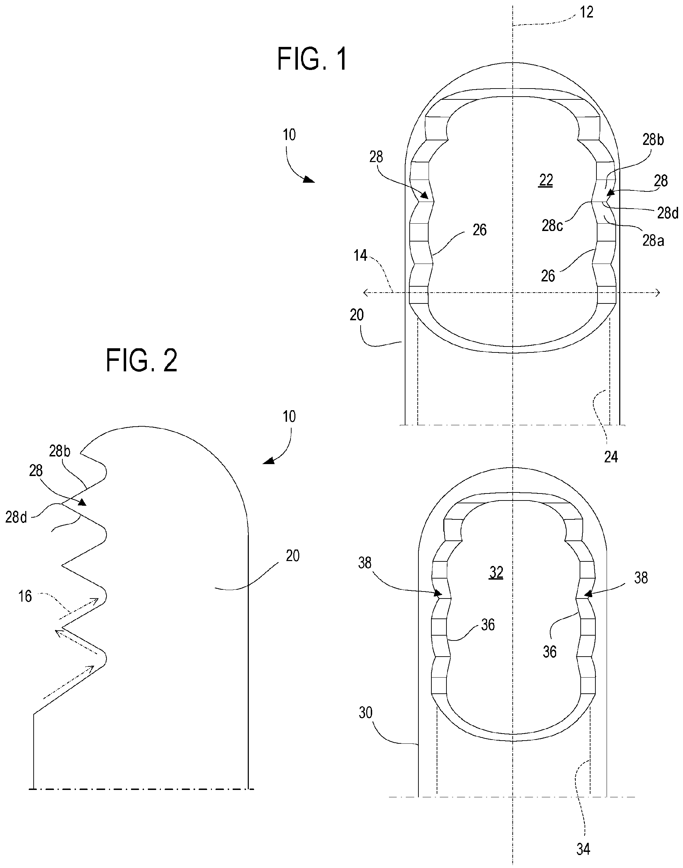

A conventional tissue shaver cutter head assembly 10 is shown in Figures 1 and 2. The cutter head assembly 10 has an outer cutter head 20 and an inner cutter head 30. The inner cutter head 30 is sized to be inserted into the outer cutter head 20, with both extending along a longitudinal axis 12. When assembled, the inner cutter head 30 can rotate, relative to the outer cutter head 20, about the longitudinal axis 12. Each cutter head 20, 30 has a respective cutter opening 22, 32 that leads into an internal cannula 24, 34 extending through the head 20, 30. Each opening 22, 32 is defined between edges 26, 36 extending along the longitudinal direction 12, and each edge 26, 36 has teeth 28, 38. As the inner cutter head 30 and outer cutter head 20 rotate relative to each other, the teeth 28, 38 of the two cutter heads slide past each other to sever adjacent tissue. The severed tissue is pulled through the inner cannula 34 for collection or disposal.

Referring for example to the illustrations of the outer cutter head 20, each tooth 28 as a proximal surface 28a and a distal surface 28b, which join at a tooth point 28c. The proximal surface 28a and distal surface 28b also form a tooth edge 28d, that extends from the tooth point 28c in a direction away from the respective opening edge 26.

The cutter opening 22, edges 26 and teeth 28 typically are formed by using a cutting tool to remove a portion of the tubular wall of the cutter head 20. The cutting tool may be a cutting wheel, a grinder, a laser cutter, or the like. The cutting tool operates along a cutting path 14 that extends perpendicular to the longitudinal axis 12, and so the proximal surface 28a, distal surface 28b, and tooth edge 28d all extend parallel to the cutting path 14, and perpendicular to the longitudinal axis 12. When the cutter head 20 has a cylindrical shape (i.e., a circular profile extending along the longitudinal axis 12), as typically is the case, the cutting path 14 defines a chord of the circular profile of the cutter head 20. Thus, in this case, the proximal surface 28a and distal surface 20b are defined by a continuous series of chord lines, and the tooth edge 28d forms a single chord line.

The teeth 38, edges 36 and opening 32 of the inner cutter head 30 typically are made like those of the outer cutter head 20 - i.e., using a cutting tool that moves along a cutting path 14 that is perpendicular to the longitudinal axis 12. Thus, the proximal and distal surfaces and tooth edge of each tooth 38 also extend parallel to the cutting path 14 and perpendicular to the longitudinal direction 12.

The foregoing construction is known to be beneficial because it allows simple and cost-effective fabrication using machine tools, such as a cutting wheel or grinder. This is particularly important in the context of shaver cutting heads, which can be very small (e.g., 3 mm or smaller in diameter, and having a wall thickness of well below 0.5 mm) and difficult to using complex machining motions. For example, a cutter head as described above can be fully formed from a tube by moving a grinding wheel back and forth along the cutting path, while indexing the tube along the longitudinal axis after each cutting tool pass (or indexing the cutting tool while holding the tube still). Each pass of the cutting tool forms a root of a tooth on each side of the opening, as well as the portion of the opening between those teeth. The shape of the grinding wheel defines the shapes of the teeth, and a full opening with all of the teeth can be formed in, for example, just four passes.

The foregoing construction is also known to be beneficial because it allows simple and cost-effective fabrication using laser cutting tools. For example, a laser cutter can be oriented to project along a cutting path that is perpendicular to the longitudinal axis, and, while maintaining a perpendicular relationship with the longitudinal axis, the laser cutter is moved along the cutter head along as shown by arrows 16 in Figure 2. As the laser cutter moves, it simultaneously cuts through the tube at two locations - i.e., one on each side of what will become the opening. At the end of the motion, the waste falls free from the remainder of the tube, leaving the opening with its edges and teeth.

With this conventional understanding in place, the concept of modifying the particular shape of the cutter head teeth has not been developed, and its benefits have remained undiscovered.

US 2006/196038 discloses an arthroscopic shaver with an inner cutting window having a plurality of teeth positioned along the lateral cutting edges, the teeth being configured for easy penetration into tissue to prevent ejection of tissue from the cutting window during closure. The inner cutting edges are formed complete by a predetermined sequence of positioning moves and grinding passes on a multi-axis grinding machine. The teeth may be symmetrically or asymmetrically placed about the tube axis when viewed in a plan view.

US 2005/065538 discloses a surgical shaver blade provided with a stationary elongated outer tube, having a cutting window at its distal tip and a rotatable elongated inner tube having a cutting window at its distal tip. Each cutting window is not symmetrical about any line in a sectional view through the window normal to the tube axis. In a preferred embodiment the cutting edges of each window have a plurality of teeth, the teeth of one lateral cutting edge being offset axially from the teeth of the other lateral edge so that the teeth of one edge align axially with the valleys between teeth on the opposite edge.

US 2019/038305 discloses surgical tool systems and methods of use thereof for performing endoscopic surgical procedures, which systems include a handpiece and a surgical accessory which detachably connects to the handpiece. The surgical accessory has a distal end which defines a cutting head incorporating two different types of tissue-treating areas.

### Summary

The invention is defined in claims 1 and 11. Further embodiments are defined in the dependent claims. In a first exemplary embodiment, there is provided a surgical tissue shaver comprising: a shaft extending from a proximal shaft end to a distal shaft end, the shaft comprising a tubular body defining a cannula extending from the proximal shaft end to the distal shaft end; and a cutter head at the distal shaft end and extending along a longitudinal axis, the cutter head having a cutter opening in fluid communication with the cannula, wherein at least a portion of the cutter opening extending along the longitudinal axis is defined between a first row of teeth and a second row of teeth opposite the first row of teeth with respect to the longitudinal axis. At least one tooth of the first row of teeth is defined by a respective proximal tooth surface and a respective distal tooth surface that intersect at a respective tooth edge, and wherein the respective tooth edge is oriented at a respective angle of less than 90° relative to the longitudinal axis.

In another exemplary embodiment, there is provided a method for manufacturing a tissue shaver cutter head, the method comprising: providing a cutter head blank comprising a hollow tube extending along a longitudinal axis; orienting a cutting tool along a first cutting path, wherein the first cutting path extends at a first angle less than 90° relative to the longitudinal axis and intersects the cutter head blank; and activating the cutting tool to cut along the first cutting path to cut a first tooth defined by a respective proximal tooth surface and a respective distal tooth surface that intersect at a respective tooth edge, wherein the respective tooth edge is oriented parallel to the first cutting path.

### Brief Description of the Drawings

Figure 1 illustrates a cutting head assembly of a conventional surgical tissue shaving instrument, shown with the inner cutting head removed from the outer cutting head.
Figure 2 is a side view of the outer cutter head of Figure 1.
Figure 3 is a schematic illustration of a surgical tissue shaving instrument according to one embodiment.
Figure 4 is a view of a first flexible shaft for a surgical tissue shaving instrument.
Figure 5 is an exploded view of a second flexible shaft for a surgical tissue shaving instrument.
Figure 6 is a front view of a cutting head having angled teeth according to one embodiment of the invention.
Figure 7 is a front view of a cutting head having angled teeth according to another embodiment of the invention.
Figure 8 is a front view of a cutting head having angled teeth according to another embodiment of the invention.
Figure 9 is a front view of a cutting head having angled teeth according to another embodiment of the invention.
Figure 10 is a front view of a cutting head having angled teeth according to another embodiment of the invention.
Figure 11 is a front view of a cutting head having angled teeth according to another embodiment of the invention.
Figure 12 is a front view of a cutting head having angled teeth according to another embodiment of the invention.
Figure 13 is a front view of a cutting head having angled teeth according to another embodiment of the invention.
Figure 14 is a side view of a cutting head having angled teeth according to another embodiment.
Figure 15 is an isometric view of a cutter head blank forming process.

### Description of Exemplary Embodiments

The following is a description of exemplary and non-limiting embodiments of a surgical tissue shaver, and parts thereof. Surgical tissue shavers are devices that might be used during arthroscopic surgical procedures to collect body tissue (bone, ligament, muscle, lesions, etc.), but the embodiments are not necessarily limited to such uses.

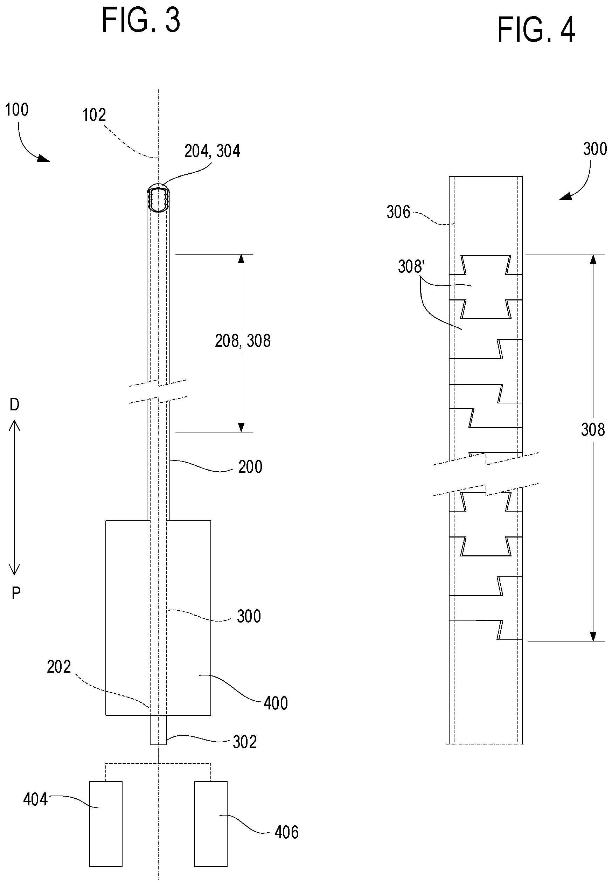

Referring now to Figures 3-5, an example of a surgical tissue shaver assembly 100 is schematically illustrated. The shaver assembly 100 includes an outer shaft 200, and an inner shaft 300 arranged concentrically within the outer shaft 200. The outer shaft extends from a proximal outer shaft end 202 to a distal outer shaft end 204, and comprises a hollow tubular body having a cannula 206 extending from the proximal outer shaft end 204 to the distal outer shaft end 206. Likewise, the inner shaft 300 extends from a proximal inner shaft end 302 to a distal inner shaft end 304, and comprises a hollow tubular body having a cannula 306 extending form the proximal inner shaft end 302 to the distal inner shaft end 304. In this case, the inner and outer distal shaft ends 204, 304 terminate at approximately the same point when the parts are assembled together. As used herein, the terms "distal" refers to a direction facing towards the patient, and "proximal" refers to a direction facing towards the person operating the instrument. The distal direction D and proximal direction P are shown by a double-headed arrow in Figure 3.

The outer shaft 200 and inner shaft 300 may include or be securable to features, such as a handle 400, a syringe 402, a drive motor 404, or the like, as known in the art.

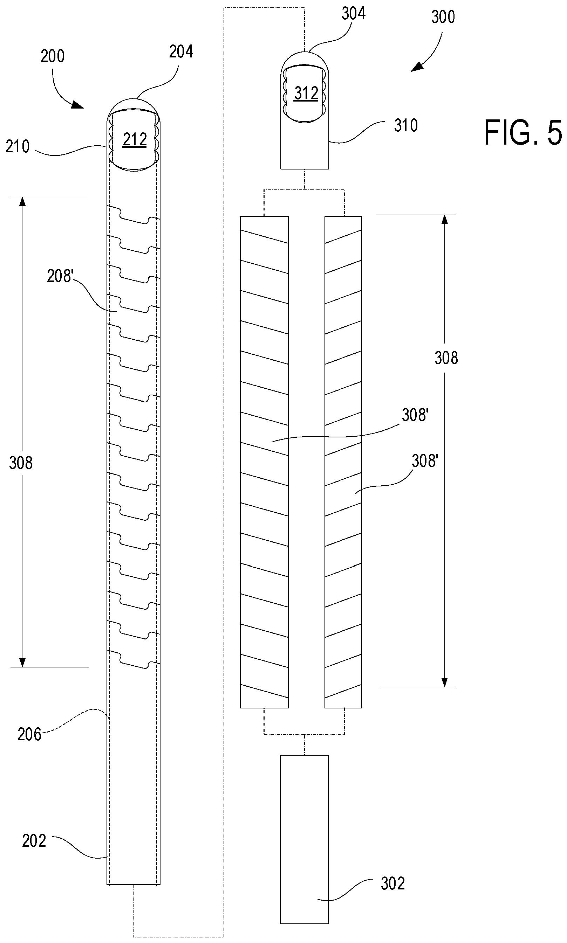

One or both of the outer shaft 200 and the inner shaft 300 may comprise respective rigid tubular structures made of surgical steel or other materials suitable for the application. The outer shaft 200 and/or inner shaft 300 also may be constructed with a flexible region 208, 308. For example, the outer shaft 200 and inner shaft 300 may have coterminous flexible regions 208, 308, such as schematically illustrated in Figure 3. Figures 4 and 5 show exemplary constructions for flexible regions 208, 308. Figure 4 shows the inner shaft 300 having a flexible region 308 formed by separate but interlocking links 308', which together form a segmented flexible tube. Such segmented flexible tube structures are described in United States Patent No. 8,366,559 and are also known in various other forms. Figure 5 shows the outer shaft 200 having a flexible region 308 being formed by a continuous helical ribbon 208' of material that forms a spring-like structure, and the inner shaft 300 being formed by concentric helical, spring-like ribbons 308'. Such structures and variation thereof, as well as alternative structures, are known in the art, and need not be described further herein.

As shown in Figure 5, the outer shaft 200 terminates at an outer cutter head 210 having an outer cutter head opening 212, and the inner shaft 300 terminates at an inner cutter head 310 having an inner cutter head opening 312. When assembled together, the outer cutter head 210 and inner cutter head 310 extend along a common longitudinal axis 102, with the outer cutter head 210 concentrically surrounding the inner cutter head 310. The outer cutter head 210 and inner cutter head 310 are rotatable relative to each other about the axis 102. In typical use, the outer cutter head 210 is held still, while the inner cutter head 310 is rotated, but this is not always the case.

When assembled together for use, the outer cutter head opening 212 and inner cutter head opening 312 are aligned along the longitudinal axis 102, such that relative rotation of the shafts 200, 300 causes the cutter head openings 212, 312 to attain different states of alignment. In the position shown in Figure 3, and Figure 5 (when assembled), the cutter head opening 212, 312 are fully aligned to form a collective opening that into the inner cannula 306. Rotation to either side causes the collective opening to become smaller, until eventually it is closed completely. At the openings 212, 312 reach the closed state, the opposite edges of the opening 212, 312 pinch and shear tissue, and deposit the tissue into the inner cannula 306. In typical use, the openings 212, 312 are reciprocated back and forth relative to each other at a very high oscillation rate, such as 5,000 to 12,000 cycles per minute, but other oscillation rates can be used. In some cases the shafts 200, 300 may be continuously rotated in one both directions (i.e., continuously rotated to open and close the collective opening more than one time, rather than reversing rotation after the collective opening closes as in normal oscillatory operation).

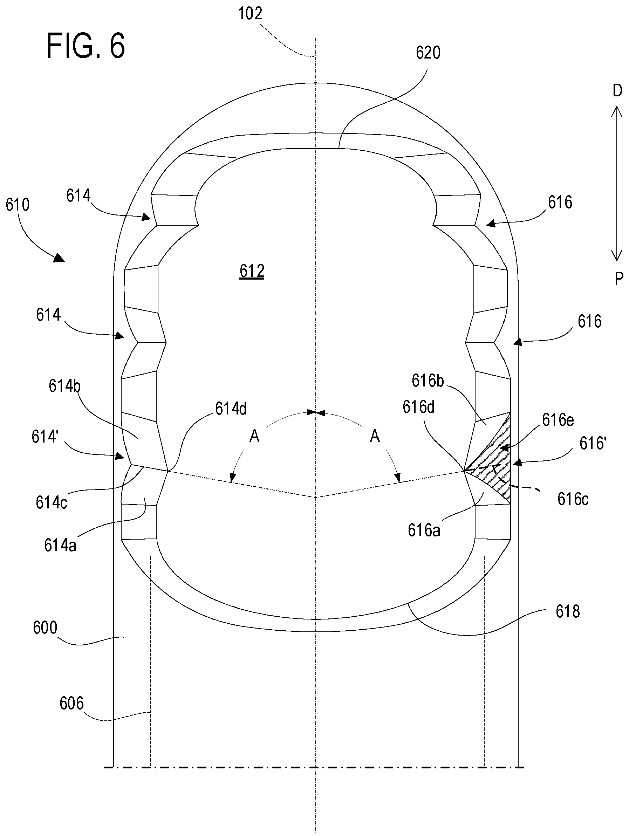

Figure 6 illustrates an example of a cutter head 610. The cutter head 610 may be an outer cutter head 210 or an inner cutter head 310. The cutter head 610 is located at the distal end of a tubular shaft 600 having a cannula 606 extending from the proximal shaft end to the distal shaft end. The cutter head 610 extends along a longitudinal axis 102, and includes a cutter head opening 612 that defines a fluid communication path from outside the cutter head 610 to the cannula 606.

The cutter opening 612 is defined, in a direction transverse to the longitudinal axis 102, between a first row of teeth 614 and a second row of teeth 616. The remainder of the cutter opening 612 may be defined, along the longitudinal axis direction, between a proximal edge 618 and a distal edge 620, but a distal edge 620 is not strictly required (i.e., the cutter head 610 may be open at the distal end).

The two rows of teeth 614, 616 are located opposite each other with respect to the longitudinal axis 102. One or more teeth 614, 616 of each row may be aligned along a line parallel to the longitudinal axis 102, but this is not required. For example, in the shown embodiment, the proximal two teeth 614, 616 of each row are arranged in parallel along the longitudinal axis 312, while the distal tooth 614, 616 of each row is not aligned with the others. The first and second rows of teeth 614, 616 may extend the full length of the cutter opening 612 (length being measured in the longitudinal axis 102), but this is not required in all cases. In the shown examples, each row has three teeth, but more or fewer teeth may be present. The number of teeth in one row also may be different from the other row.

Referring specifically to the left row of teeth 614, each tooth 614 is defined by a proximal tooth surface 614a and a distal tooth surface 614b. The proximal and distal surfaces 614a, 614b intersect each other at a tooth edge 614c. The tooth edge 614c terminates, at the inner surface of the cannula 616, at a tooth point 614d.

As will be appreciated from Figure 6, the orientation of each tooth edge 614c relative to the longitudinal axis 102 is defined by the shapes of the proximal and distal surfaces 614a, 614b. In the case of the proximal-most tooth 614', the tooth edge 614c is oriented at an angle A of less than 90° relative to the longitudinal axis 102. As understood herein, 90° to the longitudinal axis 102 refers to extending in a single imaginary plane that is orthogonal to the longitudinal axis 102, so a tooth edge 614c that is oriented at an angle A of less than 90° relative to the longitudinal axis 102 has only a single point in an single imaginary plane that is orthogonal to the longitudinal axis 102. For simplicity, the term "angled" tooth is used herein to describe at tooth having a tooth edge oriented an angle A of less than 90° relative to the longitudinal axis 102. Teeth not angled as such are referred to as "conventional" teeth.

The angle A in the embodiment of Figure 6 is an acute angle opening in the distal direction D. For purposes herein, the magnitude of the angle A is measured as viewed from a direction that is perpendicular to the longitudinal axis 102 and facing the centerline of the cutter opening 612, such as shown in Figure 6. In this case, the magnitude of the angle A may be, for example, between 45 and 85 degrees, between 75 and 85 degrees, or 80 degrees (it will be understood that, in practice, some manufacturing variables will be present that might somewhat expand the foregoing ranges and values, even if an effort is made to achieve a precise dimension of the angle A, and such variations are within the scope of the identified ranges and values).

The angled tooth 614' is expected to generate various benefits. First, the angled tooth, and especially the proximal surface 614a and/or distal surface 614b, can generate a longitudinal force along the longitudinal axis 102, which can help move tissue longitudinally. Without being bound to any theory of operation, it is believed that the proximal surface 614a tends to push tissue away from the longitudinal axis 102 because the proximal surface 614a is oriented to face generally away from the longitudinal axis 102, and the distal surface 614b tends to push tissue towards the longitudinal axis 102 because the distal surface 614b is oriented to face generally towards the longitudinal axis 102. In this case, the net result is that the severed tissue is generally driven in the distal direction D, which can help mix the severed tissue and move severed tissue back into the path of the cutting edges to be cut into smaller pieces. This can help provide a more homogenous mixture of severed tissue, and reduce large pieces that might otherwise lead to clogging problems or the like..

Another expected benefit of the angled tooth 614' is that the side edges of the tooth engage the corresponding tooth edges of the other cutter head to generate a greater shearing component to the cutting force, which can potentially decrease the force necessary to cut the tissue, and provide a cleaner cut.

Other benefits may become apparent with practice of embodiments.

Still referring to Figure 6, and particularly angled tooth 616' of the right-hand row of teeth, the angled tooth 616' optionally may be modified to remove all or some of the tooth edge. Here, tooth 616' has a proximal surface 616a and a distal surface 616b that form a virtual tooth edge 616c that extends at an angle A of less than 90° relative to the longitudinal axis 102. The virtual tooth edge 616c is an edge that would be present if the proximal and distal surfaces 616a, 616b were continued to intersect each other. The condition of having a virtual tooth edge 616c may be achieved, for example, by forming a tooth edge, then cutting away the material in the shaded region 616a. The tooth edge 616c alternatively may be partially removed, leaving a shorter tooth edge 616c, or material may be removed to also eliminate the tooth point 616d and leave a linear edge.

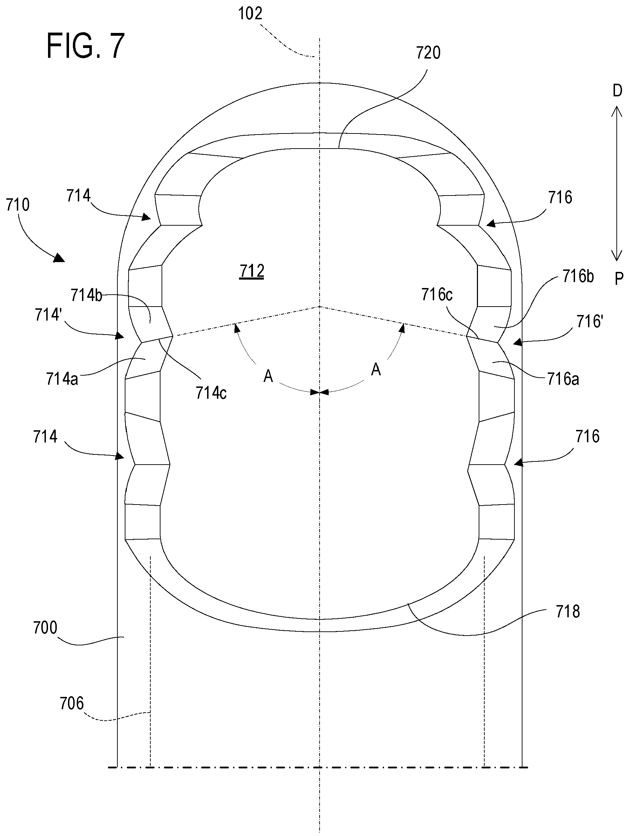

Figure 7 shows another embodiment, in which the middle tooth of each row of teeth 714, 716 is an angled tooth 714', 716'. Here, each angled teeth 714', 716' is defined by a respective proximal surface 714a, 716a and a respective distal surface 714b, 716b that form a respective tooth edge 714c, 716c oriented at an acute angle A opening in the proximal direction P. In this case, the angled teeth 714', 716' can generate a net force to drive the cut tissue in the proximal direction P, and into the cannula 606, and thus help evacuation and collection of the tissue.

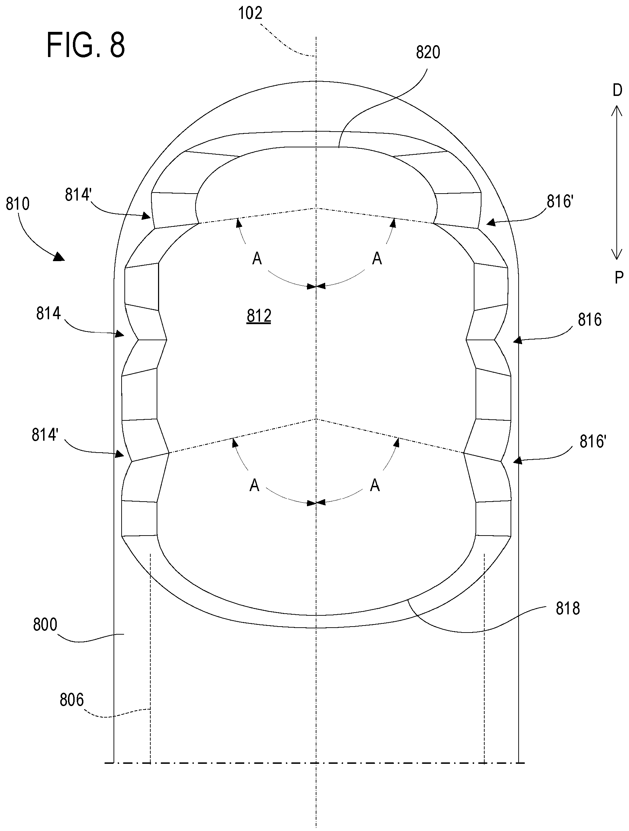

Figure 8 shows an example in which each row of teeth 814, 816 has multiple angled teeth 814', 816', and one or more conventional teeth. In this case, all of the angled teeth 814', 816' are oriented at respective acute angles A opening in the proximal direction P. Such an embodiment may be useful to adjust the magnitude of force generated along the longitudinal axis 102.

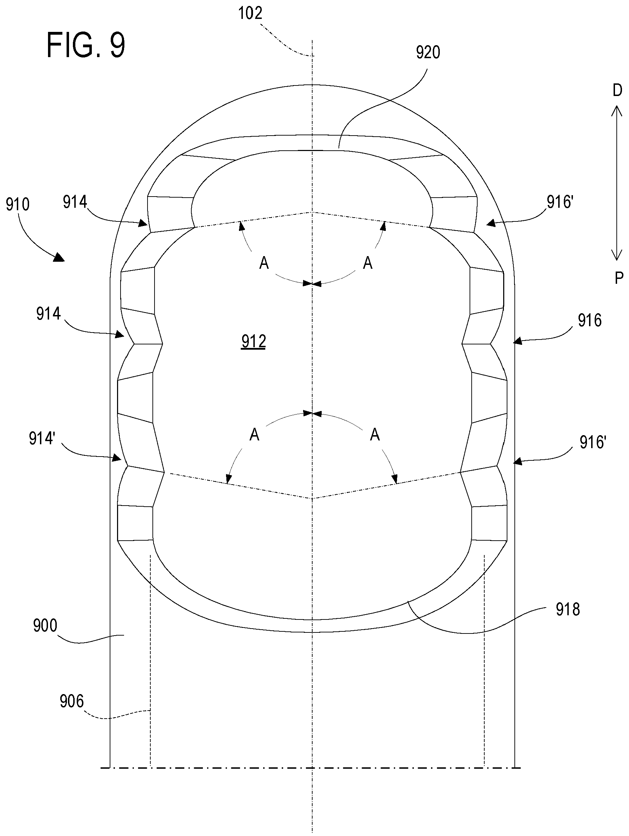

Figure 9 shows an example in which each row of teeth 914, 916 has multiple angled teeth 914', 916', and one or more conventional teeth. In this case, one opposite pair of angled teeth 914' and 916' are oriented at respective acute angles A opening in the proximal direction P, and another opposite pair of angled teeth 914' and 916' are oriented at respective acute angles A opening in the distal direction D. Such an embodiment may be useful to adjust the direction of force generated along the longitudinal axis 102 at particular locations to achieve different localized effects.

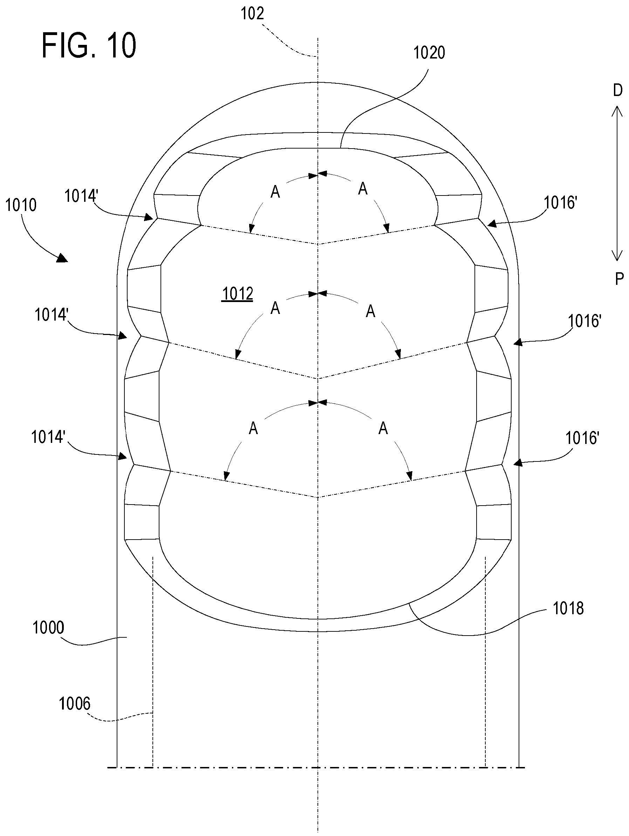

Figure 10 shows an example, in which each row of teeth 1014, 1016 entirely comprises angled teeth 1014', 1016'. In this case, all of the angled teeth 1014', 1016' are oriented at respective acute angles A opening in the proximal direction P. In other cases, all of the teeth may be oriented at acute angles A opening in the distal direction D.

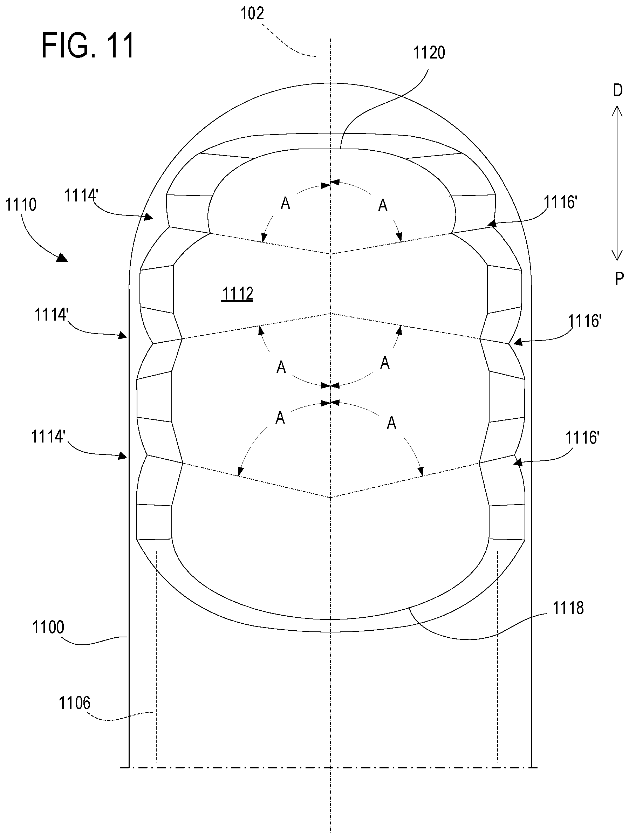

Figure 11 shows another example in which each row of teeth 1114, 1116 entirely comprises angled teeth 1114', 1116'. In this case, some opposite pairs of angled teeth 1114', 1116' are oriented at respective acute angles A opening in the proximal direction P, which other opposite pairs of angled teeth 1114', 1116' are oriented at respective angles A opening in the distal direction D.

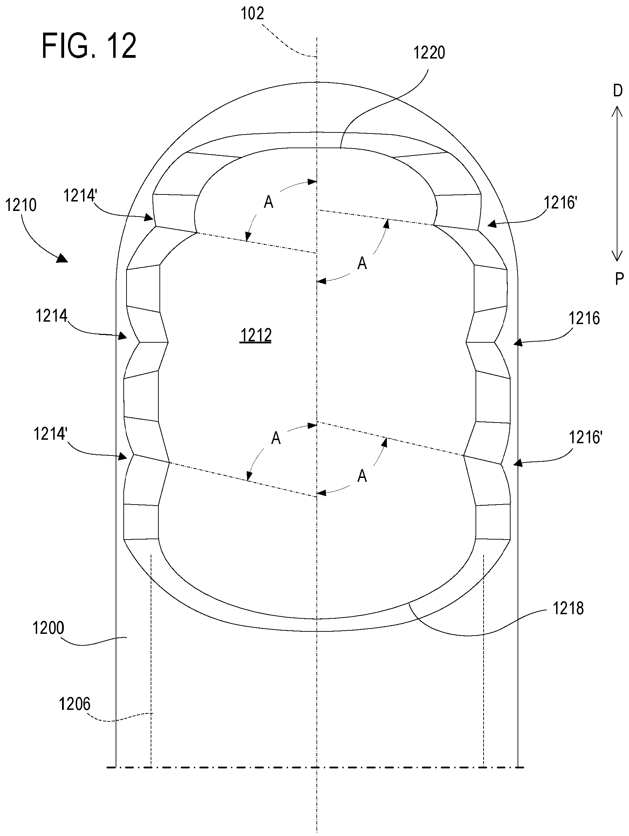

Figure 12 shows an example in which the rows of teeth 1214, 1216 include opposite pairs of angled teeth 1214', 1216' that are angled in opposite directions. Here, the distal-most angled tooth 1214' on one side is oriented at an angle A opening in the distal direction D, while the distal-most angled tooth 1216' on the other side is oriented at an angle A opening in the proximal direction P. The proximal-most angled teeth 1214', 1216' are also oriented like this. The remaining teeth may be conventional, or also may be oriented in the same way. This embodiment may provide a mixing or swirling effect within the cutter head 1210, or other potentially-beneficial movements of the severed tissue.

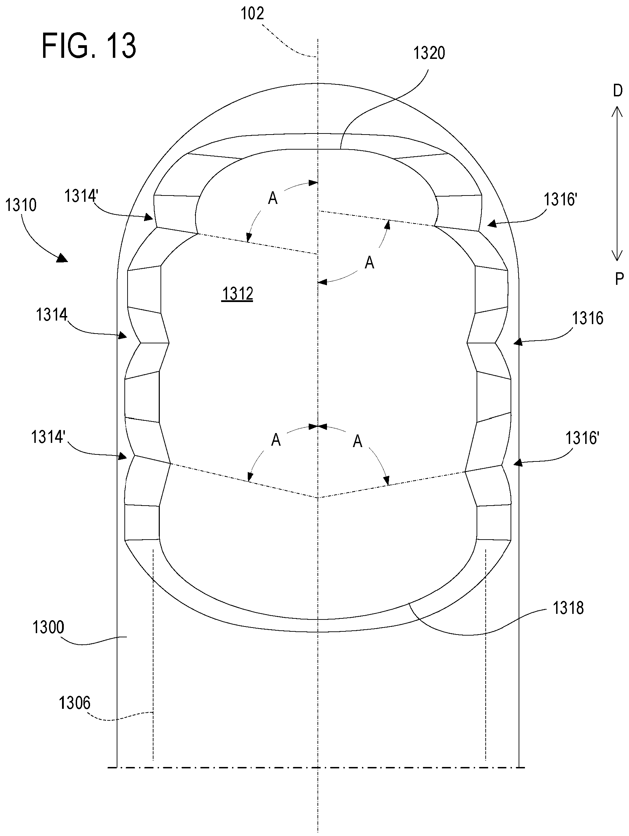

Figure 13 shows an example in which the rows of teeth 1314, 1316 include opposite pairs of angled teeth 1314', 1316' that are angled in both the same, and in opposite directions. Here, the distal-most angled tooth 1314' on one side is oriented at an angle A opening in the distal direction D, while the distal-most angled tooth 1316' on the other side is oriented at an angle A opening in the proximal direction P. In contrast, the proximal-most angled teeth 1214', 1216' are both oriented with angles A opening in the distal direction D. The remaining teeth may be conventional, or also angled.

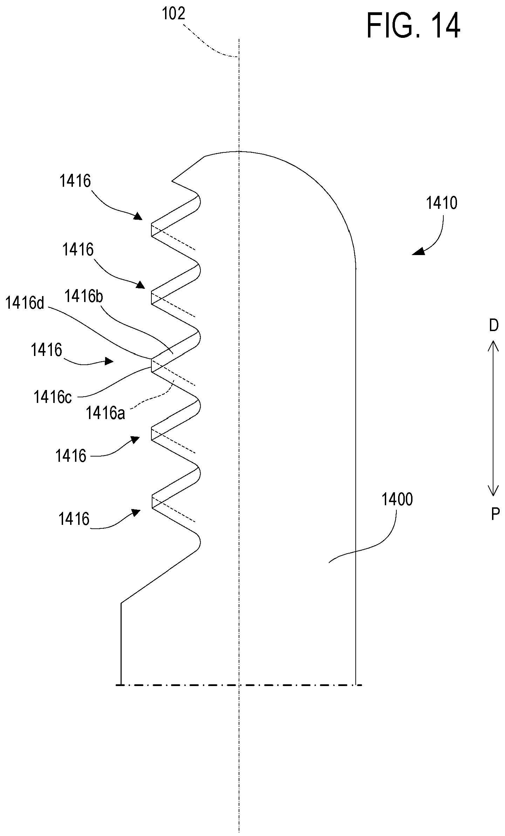

Figure 14 shows a side view of a cutting head 1410 in which there are five angled teeth 1416 in each row. As will be apparent from the positions of the distal tooth surfaces 1416b, each tooth 1416 is oriented at an angle A opening in the proximal direction P. As indicated above, this arrangement helps drive the severed tissue into the cannula in the proximal direction.

As will appreciated from the foregoing, it is anticipated that various combinations of using all angled teeth, and combinations of angled and non-angled teeth can provide various benefits. Such variations include having rows of teeth that are mirror images of each other (i.e., symmetrical about a plane parallel to the longitudinal axis 102 and intersecting the center of the cutter opening), or not. Similarly, it will be understood that angled teeth in some locations may have angles A of different magnitudes. For example, the distal angled tooth 814' may be oriented at an angle A of 80 degrees, while the proximal angled tooth 814' may be oriented at an angle A of 70 degrees. The number of teeth can also be changed to potentially gain further benefits. Other alternatives and embodiments will be apparent to persons of ordinary skill in the art in view of the present disclosure.

It will also be appreciated that the foregoing embodiments of angled teeth may be used with an inner cutter head 200, an outer cutter head 300, or both. When use only in one cutter head 200, 300, the remaining cutter head can have a conventional construction. In addition, one cutter head may have different types of angled teeth than the other. For example, an outer cutter head 200 may be provided with teeth having angles A opening distally, as shown in Figure 10, and the corresponding inner cutter head 300 may be provided with teeth having angles that open proximally. Other alternatives and embodiments will be apparent to persons of ordinary skill in the art in view of the present disclosure.

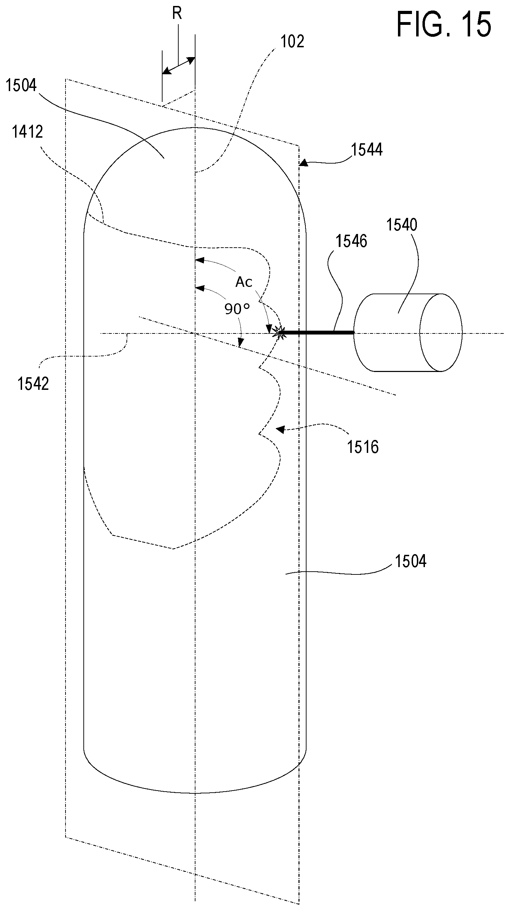

Embodiments of tissue shaver cutter heads with angled teeth may be manufactured using various methods. Figure 15 illustrates one embodiment of a method of manufacture. Here, the cutter head blank is provided as a tubular shaft 1500 having a closed hemispherical distal end 1504. A cannula (not shown) extends along the shaft 1500, as in the foregoing examples. The shaft 1500 extends along a longitudinal axis 102. The cutter head opening 1512 is shown in broken lines where the blank will be cut.

In this example, a laser cutter 1540 is provided to cut the cutter head opening 1512. The laser cutter 1540 is oriented along a cutting path 1542 that extends at an angle of less than 90° relative to the longitudinal axis and intersects the blank at the edge of the intended cutter head opening location. As shown in Figure 15, the cutting path 1542 extends at an angle Ac, which is less than 90° (compare with the illustrated 90° angle). The cutting path 1542 may lie in a plane 1544 that is offset from the centerline of the shaft 1500 (which is collinear with the longitudinal axis 102) by a radial distance R.

Once oriented, the laser cutter 1540 is activated to direct the cutting beam 1546 along the cutting path 1542. The laser cutter 1540, shaft 1500, or both, may be moved to guide the cutting beam 1546 to form the cutter head opening 1512. The cutting angle 1542, as well as the angular orientation and radial distance R of the plane 1544, may vary as the laser cutter 1540 and/or shaft 1500 moves to create the cutter head opening 1512.

As the cutting beam 1546 contacts the shaft 1500, it cuts away material to form the proximal tooth surface and the distal tooth surface of each tooth 1516. When complete, the proximal tooth surface and distal tooth surface intersect to form a tooth edge lying along the cutting direction 1542. The laser cutter 1540 can thus be used to cut the entire cutter head opening 1512, at which time the remaining waste of the tubular blank will be removed.

In the embodiment of Figure 15, the cutting angle Ac, and thus the tooth edge angle, is an acute angle opening in the proximal direction. In other cases, the cutting angle Ac and tooth edge angle may be an acute angle opening the distal direction. Other embodiments use other cutting angle orientations, such as those necessary to make the angled tooth embodiments described previously herein.

In other embodiments, the cutter head opening 1512 may be created using an abrasive grinder or other mechanical cutting device, or a combination of devices (e.g., an abrasive grinding wheel to cut the proximal and distal edges of the opening, and a laser cutter to cut the rows of teeth). Other alternatives and embodiments will be apparent to persons of ordinary skill in the art in view of the present disclosure.

It is noted that the angled cutting path Ac can add a difficulty not present in conventional cutting operations. Specifically, advancing the cutting tool along an angled cutting path Ac across the entire blank will result in asymmetrical cuts on each side of the cutter head opening 1512. This is in contrast to conventional cutting methods, in which the cutting tool is traversed across the full width of the blank along a cutting path that is perpendicular to the longitudinal axis 102, in which the cuts on both sides of the cutter head opening 1512 are identical and symmetrical.

It has been found that such undesirable asymmetrical cutting can be avoided when using a laser cutter 1540, by reducing the output and/or increasing the cutting movement speed to limit the cutting laser to penetrate the hollow blank at only a single location at any given time. This method result in some internal scarring of the cannula where the cutting laser impinges upon penetrating the opposite side of the blank, but it has been found that such scarring is not detrimental to the function of the device. In methods using mechanical cutters, undesirable asymmetrical cutting can be avoided by operating the cutter head at different angular orientations, or by rotating the blank between cuts or during cutting. Other alternatives and embodiments will be apparent to persons of ordinary skill in the art in view of the present disclosure.

The present disclosure provides a number of exemplary embodiments of the invention defined by the appended claims. The description of such embodiments is not intended to limit the scope of the claims beyond what is defined in the claims. It will also be understood that, while embodiments may provide particular advantages in certain cases, the scope of the claims is not limited to embodiments providing any particular advantage or functionality. It will further be appreciated that other embodiments encompassed by the claims may diverge from those described herein in both appearance and functionality, and the various features of particular described herein may be used with other embodiments without departing from the scope of the claims.

## Claims

1. A surgical tissue shaver comprising:
a shaft (200, 300, 600) extending from a proximal shaft end (202, 302) to a distal shaft end (204, 304), the shaft (200, 300, 600) comprising a tubular body defining a cannula (206, 306, 606) extending from the proximal shaft end (202, 302) to the distal shaft end (204, 304); and
a cutter head (210, 310, 610, 710, 810, 910, 1010, 1110, 1210, 1310, 1410) at the distal shaft end (204, 304) and extending along a longitudinal axis (102), the cutter head (210, 310, 610, 710, 810, 910, 1010, 1110, 1210, 1310, 1410) having a cutter opening (612, 712, 812, 912, 1012, 1112, 1212, 1312, 1412) in fluid communication with the cannula (206, 306, 606), wherein at least a portion of the cutter opening (612, 712, 812, 912, 1012, 1112, 1212, 1312, 1412) extending along the longitudinal axis (102) is defined between a first row of teeth (614, 714, 814, 914, 1014, 1114, 1214, 1314) and a second row of teeth (616, 716, 816, 916, 1016, 1116, 1216, 1316) opposite the first row of teeth (614, 714, 814, 914, 1014, 1114, 1214, 1314) with respect to the longitudinal axis (102);
wherein a first tooth selected from the first row of teeth (614, 714, 814, 914, 1014, 1114, 1214, 1314) is defined by a respective proximal tooth surface (614a, 616a, 714a, 716a) and a respective distal tooth surface (614b, 616b, 714b, 716b) that intersect at a respective tooth edge (614c, 616c, 714c, 716c), and wherein the respective tooth edge (614c, 616c, 714c, 716c) of the first tooth is oriented at a first angle of less than 90° relative to the longitudinal axis (102);
**characterized in that**
a second tooth selected from the first row of teeth (614, 714, 814, 914, 1014, 1114, 1214, 1314) and the second row of teeth (616, 716, 816, 916, 1016, 1116, 1216, 1316) is defined by a respective proximal tooth surface (614a, 616a, 714a, 716a) and a respective distal tooth surface (614b, 616b, 714b, 716b) that intersect at a respective tooth edge (614c, 616c, 714c, 716c), and wherein the respective tooth edge (614c, 616c, 714c, 716c) of the second tooth is oriented at a second angle relative to the longitudinal axis (102), the second angle being different from the first angle.

2. The surgical tissue shaver of claim 1, wherein the first angle is an acute angle opening in a distal direction (D) or an acute angle opening in a proximal direction (P), and the second angle is perpendicular to the longitudinal axis (102).

3. The surgical tissue shaver of claim 1, wherein at least two teeth of the first row of teeth (614, 714, 814, 914, 1014, 1114, 1214, 1314), and optionally all of the teeth of the first row of teeth (614, 714, 814, 914, 1014, 1114, 1214, 1314), is each defined by a respective proximal tooth surface (614a, 616a, 714a, 716a) and a respective distal tooth surface (614b, 616b, 714b, 716b) that intersect at a respective tooth edge (614c, 616c, 714c, 716c) oriented at a respective angle of less than 90° relative to the longitudinal axis (102).

4. The surgical tissue shaver of claim 3, wherein a respective magnitude of the respective angle of a first one of the at least two teeth is different from a respective magnitude of the respective angle of a second one of the at least two teeth.

5. The surgical tissue shaver of claim 1, wherein:
each tooth of the first row teeth, including the first tooth, is defined by a respective proximal tooth surface (614a, 616a, 714a, 716a) and a respective distal tooth surface (614b, 616b, 714b, 716b) that intersect at a respective tooth edge (614c, 616c, 714c, 716c) oriented at a respective angle of less than 90° relative to the longitudinal axis (102);
each tooth of the second row teeth, including the second tooth, is defined by a respective proximal tooth surface (614a, 616a, 714a, 716a) and a respective distal tooth surface (614b, 616b, 714b, 716b) that intersect at a respective tooth edge (614c, 616c, 714c, 716c) oriented at a respective angle of less than 90° relative to the longitudinal axis (102); and
the respective angles all are acute angles opening in a distal direction (D) or the respective angles are all acute angles opening in a proximal direction (P).

6. The surgical tissue shaver of any of claims 2 through 4, wherein:
the first angle is an acute angle opening in a distal direction (D); and
the second angle is an acute angle opening in a proximal direction (P).

7. The surgical tissue shaver of any of claims 2 through 4, wherein the first tooth and at least one other tooth selected from the first row of teeth (614, 714, 814, 914, 1014, 1114, 1214, 1314) is a mirror image of the second tooth and at least one other tooth selected from the second row of teeth (616, 716, 816, 916, 1016, 1116, 1216, 1316) with respect to the longitudinal axis (102).

8. The surgical tissue shaver of any of claims 1 through 5, wherein the second row of teeth (616, 716, 816, 916, 1016, 1116, 1216, 1316) is a mirror image of the first row of teeth (614, 714, 814, 914, 1014, 1114, 1214, 1314) with respect to the longitudinal axis (102).

9. The surgical tissue shaver of any of the preceding claims, further comprising:
a second shaft (200, 300, 600) extending from a proximal outer shaft end (202, 302) to a distal outer shaft end (204, 304), the second shaft (200, 300, 600) comprising a second tubular body defining a second cannula (206, 306, 606) extending from the proximal outer shaft end (202, 302) to the distal outer shaft end (204, 304); and
a second cutter head (210, 310, 610, 710, 810, 910, 1010, 1110, 1210, 1310, 1410) at the distal outer shaft end (204, 304) and extending along a second longitudinal axis (102), the second cutter head (210, 310, 610, 710, 810, 910, 1010, 1110, 1210, 1310, 1410) having a second cutter opening (612, 712, 812, 912, 1012, 1112, 1212, 1312, 1412) in fluid communication with the second cannula (206, 306, 606), wherein at least a portion of the second cutter opening (612, 712, 812, 912, 1012, 1112, 1212, 1312, 1412) extending along the second longitudinal axis (102) is defined between a first row of outer teeth (614, 714, 814, 914, 1014, 1114, 1214, 1314) and a second row of outer teeth (616, 716, 816, 916, 1016, 1116, 1216, 1316) opposite the first row of outer teeth (614, 714, 814, 914, 1014, 1114, 1214, 1314) with respect to the second longitudinal axis (102);
wherein the cutter head (210, 310, 610, 710, 810, 910, 1010, 1110, 1210, 1310, 1410) is concentrically received within or around the second cutter head (210, 310, 610, 710, 810, 910, 1010, 1110, 1210, 1310, 1410), with the first longitudinal axis (102) collinear with the second longitudinal axis (102), and the cutter head (210, 310, 610, 710, 810, 910, 1010, 1110, 1210, 1310, 1410) being rotatable relative to the second cutter head (210, 310, 610, 710, 810, 910, 1010, 1110, 1210, 1310, 1410) about the collinear first longitudinal axis and second longitudinal axis (102).

10. The surgical tissue shaver of any of the preceding claims, wherein the first angle is an acute angle having a magnitude of 85° to 45° , and more preferably 85° to 75°, and most preferably 80°.

11. A method for manufacturing a tissue shaver cutter head (210, 310, 610, 710, 810, 910, 1010, 1110, 1210, 1310, 1410), according to any of the preceding claims, the method comprising:
providing a cutter head blank (1500, 1504) comprising a hollow tube extending along a longitudinal axis (102);
orienting a cutting tool (1540) along a first cutting path (1542, Ac), wherein the first cutting path (1542, Ac) extends at a first angle less than 90° relative to the longitudinal axis (102) and intersects the cutter head blank (1500, 1504); and
activating the cutting tool (1540) to cut along the first cutting path (1542, Ac) to cut at least a first tooth (1516) of the first row of teeth (614, 714, 814, 914, 1014, 1114, 1214, 1314);
**characterized in that**:
the cutting tool (1540) comprises a laser, and the laser is configured to cut through an outer wall of the hollow tube at a single location at any given time.

12. The method of claim 11, further comprising activating the cutting tool (1540) to form the complete cutter opening (612, 712, 812, 912, 1012, 1112, 1212, 1312, 1412).

13. The method of claim 11, further comprising:
orienting the cutting tool (1540) along a second cutting path (1542, Ac), wherein the second cutting path (1542, Ac) extends at a second angle less than 90° relative to the longitudinal axis (102) and intersects the cutter head blank (1500, 1504);
activating the cutting tool (1540) to cut along the second cutting path (1542, Ac) to cut at least one tooth (1516) of the second row of teeth (616, 716, 816, 916, 1016, 1116, 1216, 1316).

## Patentansprüche

1. Chirurgischer Gewebe-Shaver, der Folgendes umfasst:
einen Schaft (200, 300, 600), der sich von einem proximalen Schaftende (202, 302) bis zu einem distalen Schaftende (204, 304) erstreckt, wobei der Schaft (200, 300, 600) einen röhrenförmigen Körper umfasst, der eine Kanüle (206, 306, 606) definiert, die sich von dem proximalen Schaftende (202, 302) bis zu dem distalen Schaftende (204, 304) erstreckt, und
einen Schneidkopf (210, 310, 610, 710, 810, 910, 1010, 1110, 1210, 1310, 1410) an dem distalen Schaftende (204, 304) und sich entlang einer Längsachse (102) erstreckend, wobei der Schneidkopf (210, 310, 610, 710, 810, 910, 1010, 1110, 1210, 1310, 1410) eine Schneidöffnung (612, 712, 812, 912, 1012, 1112, 1212, 1312, 1412) in Fluidverbindung mit der Kanüle (206, 306, 606) aufweist, wobei zumindest ein Abschnitt der Schneidöffnung (612, 712, 812, 912, 1012, 1112, 1212, 1312, 1412), der sich entlang der Längsachse (102) erstreckt, zwischen einer ersten Reihe von Zähnen (614, 714, 814, 914, 1014, 1114, 1214, 1314) und einer zweiten Reihe von Zähnen (616, 716, 816, 916, 1016, 1116, 1216, 1316) gegenüber der ersten Reihe von Zähnen (614, 714, 814, 914, 1014, 1114, 1214, 1314) in Bezug auf die Längsachse (102) definiert wird,
wobei ein erster Zahn, der aus der ersten Reihe von Zähnen (614, 714, 814, 914, 1014, 1114, 1214, 1314) ausgewählt ist, durch eine jeweilige proximale Zahnoberfläche (614a, 616a, 714a, 716a) und eine jeweilige distale Zahnoberfläche (614b, 616b, 714b, 716b) definiert wird, die sich an einer jeweiligen Zahnkante (614a, 616c, 714c, 716c) überschneiden, und wobei die jeweilige Zahnkante (614a, 616c, 714c, 716c) des ersten Zahns in einem ersten Winkel von weniger als 90° im Verhältnis zu der Längsachse (102) ausgerichtet ist,
**dadurch gekennzeichnet, dass**
ein zweiter Zahn, der aus der ersten Reihe von Zähnen (614, 714, 814, 914, 1014, 1114, 1214, 1314) und der zweiten Reihe von Zähnen (616, 716, 816, 916, 1016, 1116, 1216, 1316) ausgewählt ist, durch eine jeweilige proximale Zahnoberfläche (614a, 616a, 714a, 716a) und eine jeweilige distale Zahnoberfläche (614b, 616b, 714b, 716b) definiert wird, die sich an einer jeweiligen Zahnkante (614c, 616c, 714c, 716c) überschneiden, und wobei die jeweilige Zahnkante (614c, 616c, 714c, 716c) des zweiten Zahns in einem zweiten Winkel im Verhältnis zu der Längsachse (102) ausgerichtet ist, wobei sich der zweite Winkel von dem ersten Winkel unterscheidet.

2. Chirurgischer Gewebe-Shaver nach Anspruch 1, wobei der erste Winkel ein spitzer Winkel, der sich in einer distalen Richtung (D) öffnet, oder ein spitzer Winkel, der sich in einer proximalen Richtung (P) öffnet, ist und der zweite Winkel senkrecht zu der Längsachse (102) ist.

3. Chirurgischer Gewebe-Shaver nach Anspruch 1, wobei mindestens zwei Zähne der ersten Reihe von Zähnen (614, 714, 814, 914, 1014, 1114, 1214, 1314) und wahlweise alle Zähne der ersten Reihe von Zähnen (614, 714, 814, 914, 1014, 1114, 1214, 1314) jeweils durch eine jeweilige proximale Zahnoberfläche (614a, 616a, 714a, 716a) und eine jeweilige distale Zahnoberfläche (614b, 616b, 714b, 716b) definiert werden, die sich an einer jeweiligen Zahnkante (614a, 616c, 714c, 716c) überschneiden, die in einem jeweiligen Winkel von weniger als 90° im Verhältnis zu der Längsachse (102) ausgerichtet ist.

4. Chirurgischer Gewebe-Shaver nach Anspruch 3, wobei sich eine jeweilige Größe des jeweiligen Winkels eines ersten von den mindestens zwei Zähnen von einer jeweiligen Größe des jeweiligen Winkels eines zweiten von den mindestens zwei Zähnen unterscheidet.

5. Chirurgischer Gewebe-Shaver nach Anspruch 1, wobei:
jeder Zahn von den Zähnen der ersten Reihe, einschließlich des ersten Zahns, durch eine jeweilige proximale Zahnoberfläche (614a, 616a, 714a, 716a) und eine jeweilige distale Zahnoberfläche (614b, 616b, 714b, 716b) definiert wird, die sich an einer jeweiligen Zahnkante (614a, 616c, 714c, 716c) überschneiden, die in einem jeweiligen Winkel von weniger als 90° im Verhältnis zu der Längsachse (102) ausgerichtet ist,
jeder Zahn von den Zähnen der zweiten Reihe, einschließlich des zweiten Zahns, durch eine jeweilige proximale Zahnoberfläche (614a, 616a, 714a, 716a) und eine jeweilige distale Zahnoberfläche (614b, 616b, 714b, 716b) definiert wird, die sich an einer jeweiligen Zahnkante (614c, 616c, 714c, 716c) überschneiden, die in einem jeweiligen Winkel von weniger als 90° im Verhältnis zu der Längsachse (102) ausgerichtet ist, und
die jeweiligen Winkel alle spitze Winkel, die sich in einer distalen Richtung (D) öffnen, sind oder die jeweiligen Winkel alle spitze Winkel, die sich in einer proximalen Richtung (P) öffnen, sind.

6. Chirurgischer Gewebe-Shaver nach einem der Ansprüche 2 bis 4, wobei:
der erste Winkel ein spitzer Winkel, der sich in einer distalen Richtung (D) öffnet, ist und
der zweite Winkel ein spitzer Winkel, der sich in einer proximalen Richtung (P) öffnet, ist.

7. Chirurgischer Gewebe-Shaver nach einem der Ansprüche 2 bis 4, wobei der erste Zahn und mindestens ein anderer Zahn, die aus der ersten Reihe von Zähnen (614, 714, 814, 914, 1014, 1114, 1214, 1314) ausgewählt sind, ein Spiegelbild des zweiten Zahns und mindestens eines anderen Zahns, die aus der zweiten Reihe von Zähnen (616, 716, 816, 916, 1016, 1116, 1216, 1316) ausgewählt sind, in Bezug auf die Längsachse (102) sind.

8. Chirurgischer Gewebe-Shaver nach einem der Ansprüche 1 bis 5, wobei die zweite Reihe von Zähnen (616, 716, 816, 916, 1016, 1116, 1216, 1316) ein Spiegelbild der ersten Reihe von Zähnen (614, 714, 814, 914, 1014, 1114, 1214, 1314) in Bezug auf die Längsachse (102) ist.

9. Chirurgischer Gewebe-Shaver nach einem der vorhergehenden Ansprüche, der ferner Folgendes umfasst:
einen zweiten Schaft (200, 300, 600), der sich von einem proximalen äußeren Schaftende (202, 302) bis zu einem distalen äußeren Schaftende (204, 304) erstreckt, wobei der Schaft (200, 300, 600) einen zweiten röhrenförmigen Körper umfasst, der eine zweite Kanüle (206, 306, 606) definiert, die sich von dem proximalen äußeren Schaftende (202, 302) bis zu dem distalen äußeren Schaftende (204, 304) erstreckt, und
einen zweiten Schneidkopf (210, 310, 610, 710, 810, 910, 1010, 1110, 1210, 1310, 1410) an dem distalen äußeren Schaftende (204, 304) und sich entlang einer zweiten Längsachse (102) erstreckend, wobei der zweite Schneidkopf (210, 310, 610, 710, 810, 910, 1010, 1110, 1210, 1310, 1410) eine zweite Schneidöffnung (612, 712, 812, 912, 1012, 1112, 1212, 1312, 1412) in Fluidverbindung mit der zweiten Kanüle (206, 306, 606) aufweist, wobei zumindest ein Abschnitt der zweiten Schneidöffnung (612, 712, 812, 912, 1012, 1112, 1212, 1312, 1412), der sich entlang der zweiten Längsachse (102) erstreckt, zwischen einer ersten Reihe von äußeren Zähnen (614, 714, 814, 914, 1014, 1114, 1214, 1314) und einer zweiten Reihe von äußeren Zähnen (616, 716, 816, 916, 1016, 1116, 1216, 1316) gegenüber der ersten Reihe von äußeren Zähnen (614, 714, 814, 914, 1014, 1114, 1214, 1314) in Bezug auf die zweite Längsachse (102) definiert wird,
wobei der Schneidkopf (210, 310, 610, 710, 810, 910, 1010, 1110, 1210, 1310, 1410) konzentrisch innerhalb des zweiten Schneidkopfs (210, 310, 610, 710, 810, 910, 1010, 1110, 1210, 1310, 1410) oder um denselben aufgenommen wird, wobei die erste Längsachse (102) kollinear mit der zweiten Längsachse (102) ist und der Schneidkopf (210, 310, 610, 710, 810, 910, 1010, 1110, 1210, 1310, 1410) im Verhältnis zu dem zweiten Schneidkopf (210, 310, 610, 710, 810, 910, 1010, 1110, 1210, 1310, 1410) um die kollineare erste Längsachse und die zweite Längsachse (102) drehbar ist.

10. Chirurgischer Gewebe-Shaver nach einem der vorhergehenden Ansprüche, wobei der erste Winkel ein spitzer Winkel ist, der eine Größe von 85° bis 45° und bevorzugter 85° bis 75° und am bevorzugtesten 80° aufweist.

11. Verfahren zum Fertigen eines Gewebe-Shaver-Schneidkopfs (210, 310, 610, 710, 810, 910, 1010, 1110, 1210, 1310, 1410) nach einem der vorhergehenden Ansprüche, wobei das Verfahren Folgendes umfasst:
Bereitstellen eines Schneidkopfrohlings (1500, 1504), der eine hohle Röhre umfasst, die sich entlang einer Längsachse (102) erstreckt,
Ausrichten eines Schneidwerkzeugs (1540) entlang einer ersten Schneidbahn (1542, Ac), wobei sich die erste Schneidbahn (1542, Ac) in einem Winkel von weniger als 90° im Verhältnis zu der Längsachse (102) erstreckt und den Schneidkopfrohling (1500, 1504) überschneidet, und
Aktivieren des Schneidwerkzeugs (1540), um entlang der ersten Schneidbahn (1542, Ac) zu schneiden, um zumindest einen ersten Zahn (1516) der ersten Reihe von Zähnen (614, 714, 814, 914, 1014, 1114, 1214, 1314) zu schneiden,
**dadurch gekennzeichnet, dass**:
das Schneidwerkzeug (1540) einen Laser umfasst und der Laser dafür konfiguriert ist, zu jeder gegebenen Zeit an einer einzigen Position durch eine äußere Wand der hohlen Röhre zu schneiden.

12. Verfahren nach Anspruch 11, das ferner das Aktivieren des Schneidwerkzeugs (1540), um die vollständige Schneidöffnung (612, 712, 812, 912, 1012, 1112, 1212, 1312, 1412) zu formen, umfasst.

13. Verfahren nach Anspruch 11, das ferner Folgendes umfasst:
Ausrichten des Schneidwerkzeugs (1540) entlang einer zweiten Schneidbahn (1542, Ac), wobei sich die zweite Schneidbahn (1542, Ac) in einem Winkel von weniger als 90° im Verhältnis zu der Längsachse (102) erstreckt und den Schneidkopfrohling (1500, 1504) überschneidet,
Aktivieren des Schneidwerkzeugs (1540), um entlang der zweiten Schneidbahn (1542, Ac) zu schneiden, um zumindest einen Zahn (1516) der zweiten Reihe von Zähnen (616, 716, 816, 916, 1016, 1116, 1216, 1316) zu schneiden.

## Revendications

1. Rasoir chirurgical pour tissus comprenant :
un arbre (200, 300, 600) s'étendant d'une extrémité proximale (202, 302) à une extrémité distale (204, 304), l'arbre (200, 300, 600) comprenant un corps tubulaire définissant une canule (206, 306, 606) s'étendant de l'extrémité proximale de l'arbre (202, 302) à l'extrémité distale de l'arbre (204, 304) ; et
une tête de coupe (210, 310, 610, 710, 810, 910, 1010, 1110, 1210, 1310, 1410) à l'extrémité distale de l'arbre (204, 304) et s'étendant le long d'un axe longitudinal (102), la tête de coupe (210, 310, 610, 710, 810, 910, 1010, 1110, 1210, 1310, 1410) ayant une ouverture de coupe (612, 712, 812, 912, 1012, 1112, 1212, 1312, 1412) en communication fluidique avec la canule (206, 306, 606), au moins une partie de l'ouverture de coupe (612, 712, 812, 912, 1012, 1112, 1212, 1312, 1412) s'étendant le long de l'axe longitudinal (102) étant définie entre une première rangée de dents (614, 714, 814, 914, 1014, 1114, 1214, 1314) et une seconde rangée de dents (616, 716, 816, 916, 1016, 1116, 1216, 1316) opposée à la première rangée de dents (614, 714, 814, 914, 1014, 1114, 1214, 1314) par rapport à l'axe longitudinal (102) ;
une première dent sélectionnée dans la première rangée de dents (614, 714, 814, 914, 1014, 1114, 1214, 1314) étant définie par une surface de dent proximale correspondante (614a, 616a, 714a, 716a) et une surface de dent distale correspondante (614b, 616b, 714b, 716b) qui se coupent au niveau d'un bord de dent correspondant (614c, 616c, 714c, 716c), et le bord de dent correspondant (614c, 616c, 714c, 716c) de la première dent étant orienté selon un premier angle inférieur à 90° par rapport à l'axe longitudinal (102) ;
**caractérisé en ce**
**qu'**une seconde dent sélectionnée parmi la première rangée de dents (614, 714, 814, 914, 1014, 1114, 1214, 1314) et la seconde rangée de dents (616, 716, 816, 916, 1016, 1116, 1216, 1316) est définie par une surface de dent proximale correspondante (614a, 616a, 714a, 716a) et une surface de dent distale correspondante (614b, 616b, 714b, 716b) qui se coupent au niveau d'un bord de dent correspondant (614c, 616c, 714c, 716c), et le bord de dent correspondant (614c, 616c, 714c, 716c) de la seconde dent étant orienté selon un second angle par rapport à l'axe longitudinal (102), le second angle étant différent du premier angle.

2. Rasoir chirurgical pour tissus selon la revendication 1, dans lequel le premier angle est un angle aigu s'ouvrant dans une direction distale (D) ou un angle aigu s'ouvrant dans une direction proximale (P), et le second angle est perpendiculaire à l'axe longitudinal (102).

3. Rasoir chirurgical pour tissus selon la revendication 1, dans lequel au moins deux dents de la première rangée de dents (614, 714, 814, 914, 1014, 1114, 1214, 1314), et facultativement toutes les dents de la première rangée de dents (614, 714, 814, 914, 1014, 1114, 1214, 1314) sont chacune définies par une surface de dent proximale correspondante (614a, 616a, 714a, 716a) et une surface de dent distale correspondante (614b, 616b, 714b, 716b) qui se coupent au niveau d'un bord de dent correspondant (614c, 616c, 714c, 716c) orienté selon un angle correspondant inférieur à 90° par rapport à l'axe longitudinal (102).

4. Rasoir chirurgical pour tissus selon la revendication 3, dans lequel une amplitude correspondante de l'angle correspondant d'une première des au moins deux dents est différente d'une amplitude correspondante de l'angle correspondant d'une seconde des au moins deux dents.

5. Rasoir chirurgical pour tissus selon la revendication 1, dans lequel :
chaque dent de la première rangée de dents, y compris la première dent, est définie par une surface de dent proximale correspondante (614a, 616a, 714a, 716a) et une surface de dent distale correspondante (614b, 616b, 714b, 716b) qui se coupent au niveau d'un bord de dent correspondant (614c, 616c, 714c, 716c) orienté selon un angle correspondant inférieur à 90° par rapport à l'axe longitudinal (102) ;
chaque dent de la seconde rangée de dents, y compris la seconde dent, est définie par une surface de dent proximale correspondante (614a, 616a, 714a, 716a) et une surface de dent distale correspondante (614b, 616b, 714b, 716b) qui se coupent au niveau d'un bord de dent correspondant (614c, 616c, 714c, 716c) orienté selon un angle correspondant inférieur à 90° par rapport à l'axe longitudinal (102) ; et
les angles correspondants sont tous des angles aigus s'ouvrant dans une direction distale (D) ou les angles correspondants sont tous des angles aigus s'ouvrant dans une direction proximale (P).

6. Rasoir chirurgical pour tissus selon l'une quelconque des revendications 2 à 4, dans lequel :
le premier angle est un angle aigu s'ouvrant dans une direction distale (D) ; et
le second angle est un angle aigu s'ouvrant dans une direction proximale (P).

7. Rasoir chirurgical pour tissus selon l'une quelconque des revendications 2 à 4, dans lequel la première dent et au moins une autre dent sélectionnée dans la première rangée de dents (614, 714, 814, 914, 1014, 1114, 1214, 1314) sont une image miroir de la seconde dent et d'au moins une autre dent sélectionnée dans la seconde rangée de dents (616, 716, 816, 916, 1016, 1116, 1216, 1316) par rapport à l'axe longitudinal (102).

8. Rasoir chirurgical pour tissus selon l'une quelconque des revendications 1 à 5, dans lequel la seconde rangée de dents (616, 716, 816, 916, 1016, 1116, 1216, 1316) est une image miroir de la première rangée de dents (614, 714, 814,914, 1014, 1114, 1214, 1314) par rapport à l'axe longitudinal (102).

9. Rasoir chirurgical pour tissus selon l'une quelconque des revendications précédentes, comprenant en outre :
un second arbre (200, 300, 600) s'étendant d'une extrémité proximale externe de l'arbre (202, 302) à une extrémité distale externe de l'arbre (204, 304), le second arbre (200, 300, 600) comprenant un second corps tubulaire définissant une seconde canule (206, 306, 606) s'étendant de l'extrémité proximale externe de l'arbre (202, 302) à l'extrémité distale externe de l'arbre (204, 304) ; et
une seconde tête de coupe (210, 310, 610, 710, 810, 910, 1010, 1110, 1210, 1310, 1410) au niveau de l'extrémité distale externe de l'arbre (204, 304) et s'étendant le long d'un second axe longitudinal (102), la seconde tête de coupe (210, 310, 610, 710, 810, 910, 1010, 1110, 1210, 1310, 1410) ayant une seconde ouverture de coupe (612, 712, 812, 912, 1012, 1112, 1212, 1312, 1412) en communication fluidique avec la seconde canule (206, 306, 606), au moins une partie de la seconde ouverture de coupe (612, 712, 812, 912, 1012, 1112, 1212, 1312, 1412) s'étendant le long du second axe longitudinal (102) étant définie entre une première rangée de dents externes (614, 714, 814, 914, 1014, 1114, 1214, 1314) et une seconde rangée de dents externes (616, 716, 816, 916, 1016, 1116, 1216, 1316) opposée à la première rangée de dents externes (614, 714, 814, 914, 1014, 1114, 1214, 1314) par rapport au second axe longitudinal (102) ;
la tête de coupe (210, 310, 610, 710, 810, 910, 1010, 1110, 1210, 1310, 1410) étant logée concentriquement dans ou autour de la seconde tête de coupe (210, 310, 610, 710, 810, 910, 1010, 1110, 1210, 1310, 1410), le premier axe longitudinal (102) étant colinéaire au second axe longitudinal (102), et la tête de coupe (210, 310, 610, 710, 810, 910, 1010, 1110, 1210, 1310, 1410) pouvant tourner par rapport à la seconde tête de coupe (210, 310, 610, 710, 810, 910, 1010, 1110, 1210, 1310, 1410) autour du premier axe longitudinal colinéaire et du second axe longitudinal (102).

10. Rasoir chirurgical pour tissus selon l'une quelconque des revendications précédentes, dans lequel le premier angle est un angle aigu ayant une amplitude de 85° à 45°, et de manière plus préférée de 85° à 75°, et de manière la plus préférée de 80°.

11. Procédé de fabrication d'une tête de coupe de rasoir pour tissus (210, 310, 610, 710, 810, 910, 1010, 1110, 1210, 1310, 1410) selon l'une quelconque des revendications précédentes, le procédé comprenant :
la prévision d'une ébauche de tête de coupe (1500, 1504) comprenant un tube creux s'étendant le long d'un axe longitudinal (102) ;
l'orientation d'un outil de coupe (1540) le long d'un premier trajet de coupe (1542, Ac), le premier trajet de coupe (1542, Ac) s'étendant suivant un premier angle inférieur à 90° par rapport à l'axe longitudinal (102) et coupant l'ébauche de tête de coupe (1500, 1504) ; et l'activation de l'outil de coupe (1540) pour couper le long du premier trajet de coupe (1542, Ac) afin de couper au moins une première dent (1516) de la première rangée de dents (614, 714, 814, 914, 1014, 1114, 1214, 1314) ;
**caractérisé en ce que** :
l'outil de coupe (1540) comprend un laser, et que le laser est configuré pour couper à travers une paroi externe du tube creux en un seul emplacement à tout, pour un moment donné.

12. Procédé selon la revendication 11, comprenant en outre l'activation de l'outil de coupe (1540) pour former l'ouverture de coupe complète (612, 712, 812, 912, 1012, 1112, 1212, 1312, 1412).

13. Procédé selon la revendication 11, comprenant en outre :
l'orientation de l'outil de coupe (1540) le long d'un second trajet de coupe (1542, Ac), le second trajet de coupe (1542, Ac) s'étendant suivant un second angle inférieur à 90° par rapport à l'axe longitudinal (102) et coupant l'ébauche de tête de coupe (1500, 1504) ;
l'activation de l'outil de coupe (1540) pour couper le long du second trajet de coupe (1542, Ac) afin de couper au moins une dent (1516) de la seconde rangée de dents (616, 716, 816, 916, 1016, 1116, 1216, 1316).
